# EUROPEAN PATENT APPLICATION

(11) **EP 4 224 485 A1**
(43) Date of publication of application: **09.08.2023**
(21) Application number: 21891117.0
(22) Date of filing: 10.11.2021
(51) Int. Cl.: G16H 20/30

(54) **ADAPTIVE ACTION EVALUATION METHOD, ELECTRONIC DEVICE, AND STORAGE MEDIUM**

(30) Priority: 12.11.2020 CN 202011264577
(71) Applicant: Huawei Technologies Co., Ltd., Shenzhen, Guangdong 518129 (CN)
(72) Inventor: YAN, Jiabing, Shenzhen, Guangdong 518129 (CN); LIU, Hang, Shenzhen, Guangdong 518129 (CN)
(74) Representative: Thun, Clemens
(86) International application number: PCT/CN2021/129715
(87) International publication number: WO 2022/100597

(57) **Abstract**

This application discloses an adaptive action evaluation method, an electronic device, and a storage medium. The method includes: obtaining a basic information parameter of a user; obtaining a first evaluation threshold based on the basic information parameter, where the first evaluation threshold is used to provide an evaluation standard; obtaining a key evaluation parameter of a current action of the user; determining an action completion evaluation of the user based on the key evaluation parameter and the first evaluation threshold; and adjusting the first evaluation threshold based on the action completion evaluation. According to the adaptive action evaluation method, a differentiated exercise service can be provided for the user, so that the user can perform targeted physical exercise and effectively improve physical quality of the user.

## Description

### TECHNICAL FIELD

This application relates to the field of exercise and health, and in particular, to an adaptive action evaluation method, an electronic device, and a storage medium.

### BACKGROUND

In modem society, people have increasingly fast life rhythm, and have more or less physical health problems. How to maintain a good physical condition under fast-paced work becomes an urgent problem to be resolved. Currently, using a television to perform intelligent exercise guidance is a good home exercise solution. However, different individuals differ greatly in exercise capabilities, body coordination, or the like, and a user cannot be guided to perform exercise in a targeted manner.

### SUMMARY

In view of this, embodiments of this application provide an adaptive action evaluation method, an electronic device, and a storage medium, to resolve a problem that current intelligent exercise cannot guide a user to perform exercise in a targeted manner.

According to a first aspect, embodiments of this application provide an adaptive action evaluation method. First, a first evaluation threshold that has a preliminary reference effect on a physical condition of the user is obtained by using a basic information parameter of the user, to set different evaluation thresholds for physical conditions of different users, and preliminarily distinguish evaluations on user actions; and then, a specific status of currently performing exercise by the user is further reflected in real time based on a key evaluation parameter (such as a key angle and duration of completing an action) obtained when the user currently performs exercise, and evaluation is performed. The first evaluation threshold is adjusted in real time based on an evaluation result, so that the first evaluation threshold may be adjusted in real time based on a completion status of the user actions. This implements a more targeted user guidance effect.

Further, the basic information parameter may specifically include a basic body parameter, an exercise purpose, and a previous training experience of the user, or a parameter obtained by the user by performing physical fitness evaluation. It may be understood that the basic information parameter reflects a basic information overview of the user. By learning the basic information, an important evaluation standard may be provided for setting the first evaluation threshold, and evaluation thresholds of different users are preliminarily distinguished.

Further, before the user formally performs exercise, a warm-up phase may be further included. In the warm-up phase, the physical condition of the user may be further understood, to pre-adjust the first evaluation threshold. Specifically, warm-up actions may be set in a targeted manner, to determine a degree to which the user completes these warm-up actions. A higher degree indicates a better physical condition of the user. It may be understood that a warm-up completion degree of the user may be determined based on evaluation parameters (for example, angles at which the warm-up actions are completed and duration) of the warm-up actions, and the first evaluation threshold is pre-adjusted based on the warm-up completion degree. In a step of pre-adjusting the first evaluation threshold in the warm-up phase, a difference between users may be more accurately distinguished, and the user may be instructed to perform exercise in a scientific and more targeted manner.

Further, when the basic information parameter of the user is obtained, basic parameter information input by the user may be obtained through a parameter input area preset on a television interface. It may be understood that the user may input the basic information parameter of the user into a television in an active input manner. In addition to a manner in which the user actively inputs the basic parameter information through the parameter input area, the user may further receive, through a cloud server or an electronic device (such as a weight scale for measuring a body fat percentage and a smart band for measuring a heart rate) that has an associated account with the television, the basic information parameter sent by the cloud or the electronic device of the associated account in a manner in which an invoking request for the basic information parameter is initiated. Compared with a manner in which the user actively inputs the basic parameter information, a manner in which a cloud server or an electronic device of another associated account obtains the basic parameter information is more efficient, and accuracy of the manner is also ensured.

Further, the first evaluation threshold is obtained through calculation based on the basic information parameter, and is used to provide an evaluation standard. The first evaluation threshold may be obtained through calculation by using a preset algorithm. Specifically, in an optional implementation, the basic information parameter is first classified based on a preset parameter classification standard, for example, classified into a plurality of types, such as a basic body parameter, an exercise purpose, and a previous training experience. Then, a first weight is assigned to the basic information parameters of a same category, and an evaluation result of the category is obtained through performing weighted calculation based on the basic information parameters of the same category. For example, the basic body parameter may be divided into a plurality of parameters, and a first weight is assigned to a parameter of the same category, and an evaluation result of a type of the basic body parameter may be obtained through performing weighted calculation. Finally, a second weight is assigned to basic information parameters of different categories, and the first evaluation threshold is obtained through performing weighting based on evaluation results of different categories. For example, after evaluation results of the basic body parameter, the exercise purpose, and the previous training experience are respectively obtained, the second weight is assigned to the basic information parameters of the different categories, and the first evaluation threshold may be obtained by performing weighted calculation.

Further, when the user performs exercise, a corresponding monitoring module may be started based on a type of a key evaluation parameter of a current exercise action of the user, and the key evaluation parameter of the current user action is obtained by using the monitoring module. Specifically, when the user performs exercise, the television determines the type of the key evaluation parameter of the current action; if the type of the key evaluation parameter is an action angle, a photographing module is started, and the action angle of the current action is obtained by using the photographing module; or if the type of the key evaluation parameter is duration, a photographing module and a timing module are started, and the duration of the current action is obtained by using the photographing module and the timing module.

Further, the user determines, when performing exercise, that the action completion evaluation of the user based on the key evaluation parameter and the first evaluation threshold. The action completion evaluation may be performed by using a preset evaluation table, and the action completion evaluation of the user is determined based on a classification type of the basic information parameter, by using mapping information between the basic information parameter and the action completion evaluation stored in the evaluation table, and the first evaluation threshold.

Further, the user may adjust the first evaluation threshold in real time in an exercise phase based on the action completion evaluation. Specifically, determining may be performed by using a preset threshold adjustment condition. If the threshold adjustment condition is met, the first evaluation threshold is adjusted based on the threshold adjustment condition. It may be understood that with progress of the exercise performed by the user, an action of the user may be more standard, or may be less standard. In this case, the first evaluation threshold may be adjusted in real time by using the action completion evaluation, to enhance or lower an evaluation standard, and to guide the user to perform exercise in a more targeted manner.

According to a second aspect, embodiments of this application provide an electronic device with a screen, including a memory, a processor, and a computer program stored in the memory and executable on the processor, where when executing the computer program, the processor implements the following steps.

First, a first evaluation threshold that has a preliminary reference effect on a physical condition of the user is obtained by using a basic information parameter of the user, to set different evaluation thresholds for physical conditions of different users, and preliminarily distinguish evaluations on user actions; and then, a specific status of currently performing exercise by the user is further reflected in real time based on a key evaluation parameter (such as a key angle and duration of completing an action) obtained when the user currently performs exercise, and evaluation is performed. The first evaluation threshold is adjusted in real time based on an evaluation result, so that the first evaluation threshold may be adjusted in real time based on a completion status of the user actions. This implements a more targeted user guidance effect.

The basic information parameter may specifically include a basic body parameter, an exercise purpose, and a previous training experience of the user, or a parameter obtained by the user by performing physical fitness evaluation. It may be understood that the basic information parameter reflects a basic information overview of the user. By learning the basic information, an important evaluation standard may be provided for setting the first evaluation threshold, and evaluation thresholds of different users are preliminarily distinguished.

Further, before the user formally performs exercise, a warm-up phase may be further included. In the warm-up phase, the physical condition of the user may be further understood, to pre-adjust the first evaluation threshold. Specifically, warm-up actions may be set in a targeted manner, to determine a degree to which the user completes these warm-up actions. A higher degree indicates a better physical condition of the user. It may be understood that a warm-up completion degree of the user may be determined based on evaluation parameters (for example, angles at which the warm-up actions are completed and duration) of the warm-up actions, and the first evaluation threshold is pre-adjusted based on the warm-up completion degree. In a step of pre-adjusting the first evaluation threshold in the warm-up phase, a difference between users may be more accurately distinguished, and the user may be instructed to perform exercise in a scientific and more targeted manner.

Further, when the basic information parameter of the user is obtained, basic parameter information input by the user may be obtained through a parameter input area preset on a television interface. It may be understood that the user may input the basic information parameter of the user into a television in an active input manner. In addition to a manner in which the user actively inputs the basic parameter information through the parameter input area, the user may further receive, through a cloud server or an electronic device (such as a weight scale for measuring a body fat percentage and a smart band for measuring a heart rate) that has an associated account with the television, the basic information parameter sent by the cloud or the electronic device of the associated account in a manner in which an invoking request for the basic information parameter is initiated. Compared with a manner in which the user actively inputs the basic parameter information, a manner in which a cloud server or an electronic device of another associated account obtains the basic parameter information is more efficient, and accuracy of the manner is also ensured.

Further, the first evaluation threshold is obtained through calculation based on the basic information parameter, and is used to provide an evaluation standard. The first evaluation threshold may be obtained through calculation by using a preset algorithm. Specifically, in an optional implementation, the basic information parameter is first classified based on a preset parameter classification standard, for example, classified into a plurality of types, such as a basic body parameter, an exercise purpose, and a previous training experience. Then, a first weight is assigned to the basic information parameters of a same category, and an evaluation result of the category is obtained through performing weighted calculation based on the basic information parameters of the same category. For example, the basic body parameter may be divided into a plurality of parameters, and a first weight is assigned to a parameter of the same category, and an evaluation result of a type of the basic body parameter may be obtained through performing weighted calculation. Finally, a second weight is assigned to basic information parameters of different categories, and the first evaluation threshold is obtained through performing weighting based on evaluation results of different categories. For example, after evaluation results of the basic body parameter, the exercise purpose, and the previous training experience are respectively obtained, the second weight is assigned to the basic information parameters of the different categories, and the first evaluation threshold may be obtained by performing weighted calculation.

Further, when the user performs exercise, a corresponding monitoring module may be started based on a type of a key evaluation parameter of a current exercise action of the user, and the key evaluation parameter of the current user action is obtained by using the monitoring module. Specifically, when the user performs exercise, the television determines the type of the key evaluation parameter of the current action; if the type of the key evaluation parameter is an action angle, a photographing module is started, and the action angle of the current action is obtained by using the photographing module; or if the type of the key evaluation parameter is duration, a photographing module and a timing module are started, and the duration of the current action is obtained by using the photographing module and the timing module.

Further, the user determines, when performing exercise, that the action completion evaluation of the user based on the key evaluation parameter and the first evaluation threshold. The action completion evaluation may be performed by using a preset evaluation table, and the action completion evaluation of the user is determined based on a classification type of the basic information parameter, by using mapping information between the basic information parameter and the action completion evaluation stored in the evaluation table, and the first evaluation threshold.

Further, the user may adjust the first evaluation threshold in real time in an exercise phase based on the action completion evaluation. Specifically, determining may be performed by using a preset threshold adjustment condition. If the threshold adjustment condition is met, the first evaluation threshold is adjusted based on the threshold adjustment condition. It may be understood that with progress of the exercise performed by the user, an action of the user may be more standard, or may be less standard. In this case, the first evaluation threshold may be adjusted in real time by using the action completion evaluation, to enhance or lower an evaluation standard, and to guide the user to perform exercise in a more targeted manner.

According to a third aspect, embodiments of this application provide a computer-readable storage medium, including a computer program, where when the computer program is executed by a processor, steps of the method according to the first aspect are implemented.

In embodiments of this application, the first evaluation threshold is set by obtaining the basic information parameter of the user, so that a more proper evaluation standard may be set based on physical conditions of different users. When the user performs exercise, through obtaining the key evaluation parameter of the current user action, and the first evaluation threshold that is set, the action completion evaluation of the user is determined, to flexibly adjust the first evaluation threshold based on an actual completion status of the user action. In embodiments of this application, a differentiated exercise service can be provided for the user, so that the user can perform targeted physical exercise and effectively improve physical quality of the user.

### BRIEF DESCRIPTION OF DRAWINGS

To describe technical solutions in embodiments of this application more clearly, the following briefly describes the accompanying drawings for embodiments. It is clear that the accompanying drawings in the following descriptions show merely some embodiments of this application, and a person of ordinary skill in the art may derive other drawings from these accompanying drawings without creative efforts.
FIG. 1 is a schematic diagram of a structure of an electronic device according to an embodiment of this application;
FIG. 2 is a block diagram of a software structure of an electronic device according to an embodiment of this application;
FIG. 3a is a schematic diagram of implementing information entry through television interface interaction according to an embodiment of this application;
FIG. 3b is still another schematic diagram of implementing information entry through television interface interaction according to an embodiment of this application;
FIG. 3c is a schematic diagram of implementing physical fitness evaluation through television interface interaction according to an embodiment of this application;
FIG. 4a is a schematic diagram of entering a basic body parameter of a user through a television interface according to an embodiment of this application;
FIG. 4b is a schematic diagram of displaying a connectable smart band on a television interface according to an embodiment of this application;
FIG. 4c is a schematic diagram of prompting a user to perform a heart rate measurement operation by using a connected smart band on a television interface according to an embodiment of this application;
FIG. 4d is a schematic diagram of entering an exercise purpose of a user through a television interface;
FIG. 4e is a schematic diagram of entering a previous training experience of a user through a television interface according to an embodiment of this application;
FIG. 4f is a schematic diagram of selecting a training item through an interface according to an embodiment of this application;
FIG. 4g is another schematic diagram of selecting a training item through an interface according to an embodiment of this application;
FIG. 5 is a schematic diagram of entering a warm-up scenario through a television interface according to an embodiment of this application;
FIG. 6a is a schematic diagram of guiding a user to perform a warm-up action according to an embodiment of this application;
FIG. 6b is another schematic diagram of guiding a user to perform a warm-up action according to an embodiment of this application;
FIG. 7a is a schematic diagram of recognizing an inclination angle by using a plurality of photographing devices when a user performs a head stretching warm-up action according to an embodiment of this application;
FIG. 7b is another schematic diagram of recognizing an inclination angle by using a plurality of photographing devices when a user performs a head stretching warm-up action according to an embodiment of this application;
FIG. 8a is a schematic diagram of a piece of action correction reminding information according to an embodiment of this application;
FIG. 8b is another schematic diagram of a piece of action correction reminding information according to an embodiment of this application;
FIG. 8c is still another schematic diagram of a piece of action correction reminding information according to an embodiment of this application;
FIG. 9a is a schematic interface diagram of an adaptive and iterative action evaluation standard according to an embodiment of this application; and
FIG. 9b is a schematic interface diagram of another adaptive and iterative action evaluation standard according to an embodiment of this application.

### DESCRIPTION OF EMBODIMENTS

The following describes the technical solutions in embodiments of this application with reference to the accompanying drawings in embodiments of this application. In description in embodiments of this application, "/" means "or" unless otherwise specified. For example, A/B may represent A or B. In this specification, "and/or" describes only an association relationship for describing associated objects and represents that three relationships may exist. For example, A and/or B may represent the following three cases: Only A exists, both A and B exist, and only B exists.

The terms "first" and "second" mentioned below are merely intended for a purpose of description, and shall not be understood as an indication or implication of relative importance or implicit indication of the number of indicated technical features. Therefore, a feature restricted by "first" or "second" may explicitly or implicitly include one or more features. In descriptions of embodiments of this application, "a plurality of" means two or more unless otherwise specified.

In embodiments of this application, the word "example" or "for example" is used to represent giving an example, an illustration, or a description. Any embodiment or design scheme described as "example" or "for example" in embodiments of this application should not be explained as being more preferred or having more advantages than another embodiment or design scheme. Exactly, use of the word such as "example" or "for example" is intended to present a relative concept in a specific manner.

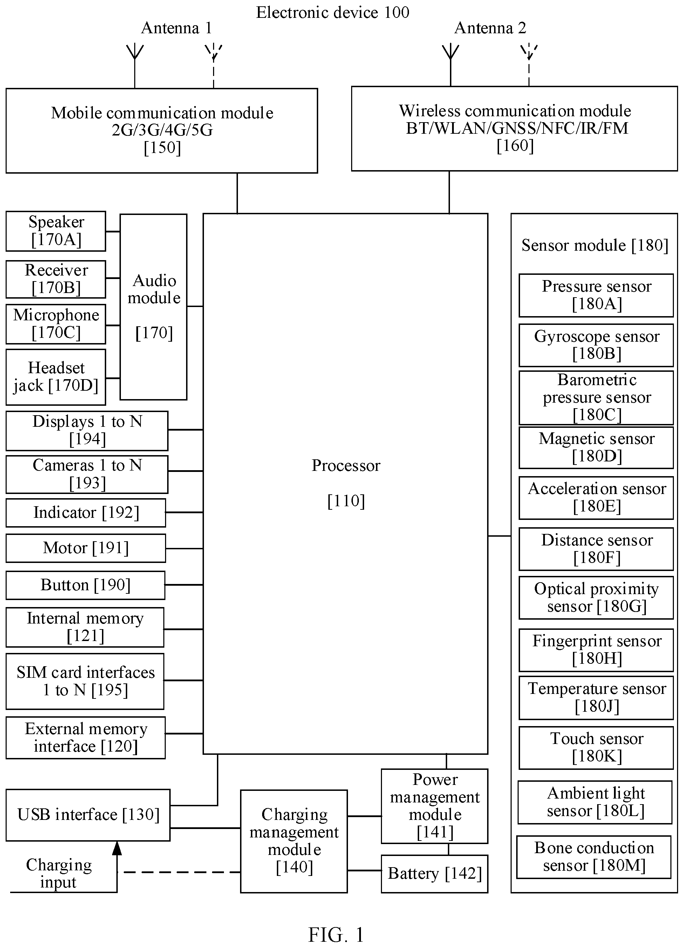

FIG. 1 is a schematic diagram of a structure of an electronic device 100.

The electronic device 100 may include a processor 110, an external memory interface 120, an internal memory 121, a universal serial bus (universal serial bus, USB) interface 130, a charging management module 140, a power management module 141, a battery 142, an antenna 1, an antenna 2, a mobile communication module 150, a wireless communication module 160, an audio module 170, a speaker 170A, a receiver 170B, a microphone 170C, a headset jack 170D, a sensor module 180, a button 190, a motor 191, an indicator 192, a camera 193, a display 194, a subscriber identity module (subscriber identity module, SIM) card interface 195, and the like. The sensor module 180 may include a pressure sensor 180A, a gyroscope sensor 180B, a barometric pressure sensor 180C, a magnetic sensor 180D, an acceleration sensor 180E, a distance sensor 180F, an optical proximity sensor 180G, a fingerprint sensor 180H, a temperature sensor 180J, a touch sensor 180K, an ambient light sensor 180L, a bone conduction sensor 180M, and the like.

It may be understood that the structure shown in embodiments of this application does not constitute a specific limitation on the electronic device 100. In some other embodiments of this application, the electronic device 100 may include more or fewer components than those shown in the figure, some components may be combined, or some components may be split, or different component arrangements may be used. The components shown in the figure may be implemented by hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, a neural-network processing unit (neural-network processing unit, NPU), and/or the like. Different processing units may be independent components, or may be integrated into one or more processors.

The controller may generate an operation control signal based on an instruction operation code and a time sequence signal, to control instruction reading and instruction execution.

A memory may be further disposed in the processor 110, and is configured to store instructions and data. In some embodiments, the memory in the processor 110 is a cache memory. The memory may store instructions or data that has been used or cyclically used by the processor 110. If the processor 110 needs to use the instructions or the data again, the processor may directly invoke the instructions or the data from the memory. This avoids repeated access, reduces waiting time of the processor 110, and improves system efficiency.

In some embodiments, the processor 110 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) port, and/or the like.

The I2C interface is a two-way synchronization serial bus, and includes one serial data line (serial data line, SDA) and one serial clock line (serial clock line, SCL). In some embodiments, the processor 110 may include a plurality of groups of I2C buses. The processor 110 may be separately coupled to the touch sensor 180K, a charger, a flash, the camera 193, and the like through different I2C bus interfaces. For example, the processor 110 may be coupled to the touch sensor 180K through the I2C interface, so that the processor 110 communicates with the touch sensor 180K through the I2C bus interface, to implement a touch function of the electronic device 100.

The I2S interface may be configured to perform audio communication. In some embodiments, the processor 110 may include a plurality of groups of I2S buses. The processor 110 may be coupled to the audio module 170 through the I2S bus, to implement communication between the processor 110 and the audio module 170. In some embodiments, the audio module 170 may transmit an audio signal to the wireless communication module 160 through the I2S interface, to implement a function of answering a call through a Bluetooth headset.

The PCM interface may also be used to perform audio communication, and sample, quantize, and code an analog signal. In some embodiments, the audio module 170 may be coupled to the wireless communication module 160 through a PCM bus interface. In some embodiments, the audio module 170 may also transmit an audio signal to the wireless communication module 160 through the PCM interface, to implement a function of answering a call through a Bluetooth headset. Both the I2S interface and the PCM interface may be configured to perform the audio communication.

The UART interface is a universal serial data bus, and is configured to perform asynchronous communication. The bus may be a two-way communication bus. The bus converts to-be-transmitted data between serial communication and parallel communication. In some embodiments, the UART interface is usually configured to connect the processor 110 to the wireless communication module 160. For example, the processor 110 communicates with a Bluetooth module in the wireless communication module 160 through the UART interface, to implement a Bluetooth function. In some embodiments, the audio module 170 may transmit an audio signal to the wireless communication module 160 through the UART interface, to implement a function of playing music through a Bluetooth headset.

The MIPI interface may be configured to connect the processor 110 to a peripheral component such as the display 194 or the camera 193. The MIPI interface includes a camera serial interface (camera serial interface, CSI), a display serial interface (display serial interface, DSI), and the like. In some embodiments, the processor 110 communicates with the camera 193 via the CSI, to implement a photographing function of the electronic device 100. The processor 110 communicates with the display 194 via the DSI interface, to implement a display function of the electronic device 100.

The GPIO interface may be configured by software. The GPIO interface may be configured as a control signal or a data signal. In some embodiments, the GPIO interface may be configured to connect the processor 110 to the camera 193, the display 194, the wireless communication module 160, the audio module 170, the sensor module 180, or the like. The GPIO interface may alternatively be configured as an I2C interface, an I2S interface, a UART interface, an MIPI interface, or the like.

The USB interface 130 is an interface that conforms to a USB standard specification, and may be specifically a mini USB port, a micro USB port, a USB Type C port, or the like. The USB interface 130 may be configured to connect to a charger to charge the electronic device 100, or may be configured to transmit data between the electronic device 100 and a peripheral device, or may be configured to connect to a headset for playing audio through the headset. Alternatively, the port may be configured to connect to another electronic device, for example, an AR device.

It may be understood that an interface connection relationship between the modules shown in this embodiment of this application is merely an example for description, and constitutes no limitation on the structure of the electronic device 100. In some other embodiments of this application, the electronic device 100 may alternatively use an interface connection manner different from an interface connection manner in this embodiment, or use a combination of a plurality of interface connection manners.

The charging management module 140 is configured to receive a charging input from the charger. The charger may be a wireless charger or a wired charger. In some embodiments in which wired charging is used, the charging management module 140 may receive a charging input from the wired charger through the USB interface 130. In some embodiments in which wireless charging is used, the charging management module 140 may receive a wireless charging input through a wireless charging coil of the electronic device 100. The charging management module 140 may further supply power to the electronic device by using the power management module 141 when the battery 142 is charged.

The power management module 141 is configured to connect to the battery 142, the charging management module 140, and the processor 110. The power management module 141 receives an input from the battery 142 and/or the charging management module 140, and supplies power to the processor 110, the internal memory 121, the display 194, the camera 193, the wireless communication module 160, and the like. The power management module 141 may be configured to monitor parameters such as a battery capacity, a battery cycle count, and a battery state of health (electric leakage and impedance). In some other embodiments, the power management module 141 may alternatively be disposed in the processor 110. In some other embodiments, the power management module 141 and the charging management module 140 may alternatively be disposed in a same device.

A wireless communication function of the electronic device 100 may be implemented by using the antenna 1, the antenna 2, the mobile communication module 150, the wireless communication module 160, the modem processor, the baseband processor, and the like.

The antenna 1 and the antenna 2 are configured to transmit and receive an electromagnetic wave signal. Each antenna in the electronic device 100 may be configured to cover one or more communication frequency bands. Different antennas may be multiplexed to improve antenna utilization. For example, the antenna 1 may be multiplexed as a diversity antenna of a wireless local area network. In some other embodiments, the antennas may be used in combination with a tuning switch.

The mobile communication module 150 may provide a wireless communication solution that is applied to the electronic device 100 and that includes 2G/3G/4G/5G. The mobile communication module 150 may include at least one filter, a switch, a power amplifier, a low noise amplifier (low noise amplifier, LNA), and the like. The mobile communication module 150 may receive an electromagnetic wave by using the antenna 1, perform processing such as filtering or amplification on the received electromagnetic wave, and transmit the electromagnetic wave to the modem processor for demodulation. The mobile communication module 150 may further amplify a signal modulated by the modem processor, and convert the signal into an electromagnetic wave for radiation through the antenna 1. In some embodiments, at least some functional modules of the mobile communication module 150 may be disposed in the processor 110. In some embodiments, at least some function modules in the mobile communication module 150 may be disposed in a same component as at least some modules in the processor 110.

The modem processor may include a modulator and a demodulator. The modulator is configured to modulate a to-be-sent low-frequency baseband signal into a medium-high frequency signal. The demodulator is configured to demodulate the received electromagnetic wave signal into a low-frequency baseband signal. Then, the demodulator transmits the low frequency baseband signal obtained through demodulation to the baseband processor for processing. After being processed by the baseband processor, the low frequency baseband signal is transmitted to the application processor. The application processor outputs a sound signal by using an audio device (which is not limited to the speaker 170A, the receiver 170B, or the like), or displays an image or a video by using the display 194. In some embodiments, the modem processor may be an independent device. In some other embodiments, the modem processor may be independent of the processor 110, and is disposed in a same component as the mobile communication module 150 or another function module.

The wireless communication module 160 may provide a wireless communication solution that is applied to the electronic device 100, and that includes a wireless local area network (wireless local area networks, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like. The wireless communication module 160 may be one or more components integrating at least one communication processing module. The wireless communication module 160 receives an electromagnetic wave through the antenna 2, performs frequency modulation and filtering processing on an electromagnetic wave signal, and sends a processed signal to the processor 110. The wireless communication module 160 may further receive a to-be-sent signal from the processor 110, perform frequency modulation and amplification on the signal, and convert the signal into an electromagnetic wave for radiation through the antenna 2.

In some embodiments, in the electronic device 100, the antenna 1 and the mobile communication module 150 are coupled, and the antenna 2 and the wireless communication module 160 are coupled, so that the electronic device 100 can communicate with a network and another device by using a wireless communication technology. The wireless communication technology may include a global system for mobile communications (global system for mobile communications, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TD-SCDMA), long term evolution (long term evolution, LTE), BT, a GNSS, a WLAN, NFC, FM, an IR technology, and/or the like. The GNSS may include a global positioning system (global positioning system, GPS), a global navigation satellite system (global navigation satellite system, GLONASS), a BeiDou navigation satellite system (beidou navigation satellite system, BDS), a quasi-zenith satellite system (quasi-zenith satellite system, QZSS), and/or a satellite based augmentation system (satellite based augmentation systems, SBAS).

The electronic device 100 may implement a display function through the GPU, the display 194, the application processor, and the like. The GPU is a microprocessor for image processing, and is connected to the display 194 and the application processor. The GPU is configured to: perform mathematical and geometric computation, and render an image. The processor 110 may include one or more GPUs that execute program instructions to generate or change display information.

The display 194 is configured to display an image, a video, and the like. The display 194 includes a display panel. The display panel may be a liquid crystal display (liquid crystal display, LCD), an organic light-emitting diode (organic light-emitting diode, OLED), an active-matrix organic light emitting diode (active-matrix organic light emitting diode, AMOLED), a flexible light-emitting diode (flexible light-emitting diode, FLED), a mini-LED, a micro-LED, a micro-OLED, a quantum dot light emitting diode (quantum dot light emitting diode, QLED), or the like. In some embodiments, the electronic device 100 may include one or N displays 194, where N is a positive integer greater than 1.

The electronic device 100 may implement a photographing function through the camera 193, the ISP, the video codec, the GPU, the display 194, the application processor and the like.

The ISP is configured to process data fed back by the camera 193. For example, during shooting, a shutter is pressed, light is transmitted to a photosensitive element of the camera through a lens, an optical signal is converted into an electrical signal, and the photosensitive element of the camera transmits the electrical signal to the ISP for processing, to convert the electrical signal into a visible image. The ISP may further perform algorithm optimization on noise, brightness, and complexion of the image. The ISP may further optimize parameters such as exposure and color temperature of a photographing scenario. In some embodiments, the ISP may be disposed in the camera 193.

The camera 193 is configured to capture a static image or a video. An optical image of an object is generated through the lens, and is projected onto the photosensitive element. The photosensitive element may be a charge coupled device (charge coupled device, CCD) or a complementary metal-oxide-semiconductor (complementary metal-oxide-semiconductor, CMOS) phototransistor. The photosensitive element converts an optical signal into an electrical signal, and then transmits the electrical signal to the ISP for converting the electrical signal into a digital image signal. The ISP outputs the digital image signal to the DSP for processing. The DSP converts the digital image signal into an image signal in a standard format such as an RGB format or a YUV format. In some embodiments, the electronic device 100 may include one or N cameras 193, where N is a positive integer greater than 1.

The digital signal processor is configured to process a digital signal, and may process another digital signal in addition to the digital image signal. For example, when the electronic device 100 selects a frequency, the digital signal processor is configured to perform Fourier transform on frequency energy.

The video codec is configured to: compress or decompress a digital video. The electronic device 100 may support one or more video codecs. In this way, the electronic device 100 may play back or record videos in a plurality of coding formats, for example, moving picture experts group (moving picture experts group, MPEG)1, MPEG2, MPEG3, and MPEG4.

The NPU is a neural-network (neural-network, NN) computing processor, quickly processes input information by referring to a structure of a biological neural network, for example, by referring to a mode of transmission between human brain neurons, and may further continuously perform self-learning. Applications such as intelligent cognition of the electronic device 100 may be implemented through the NPU, for example, image recognition, facial recognition, speech recognition, and text understanding.

The external memory interface 120 may be configured to connect to an external memory card, for example, a micro SD card, to extend a storage capability of the electronic device 100. The external memory card communicates with the processor 110 through the external memory interface 120, to implement a data storage function. For example, files such as music and a video are stored in the external storage card.

The internal memory 121 may be configured to store computer-executable program code. The executable program code includes instructions. The internal memory 121 may include a program storage area and a data storage area. The program storage area may store an operating system, an application required by at least one function (for example, a voice play function and an image play function), and the like. The data storage area may store data (such as audio data and an address book) created during use of the electronic device 100, and the like. In addition, the internal memory 121 may include a high-speed random access memory, and may further include a nonvolatile memory, for example, at least one magnetic disk storage device, a flash storage device, a universal flash storage (universal flash storage, UFS), and the like. The processor 110 runs instructions stored in the internal memory 121 and/or instructions stored in the memory disposed in the processor, to perform various function applications and data processing of the electronic device 100.

The electronic device 100 may implement an audio function, for example, music playing and recording, through the audio module 170, the speaker 170A, the receiver 170B, the microphone 170C, the headset jack 170D, the application processor, and the like.

The audio module 170 is configured to convert digital audio information into an analog audio signal for output, and is also configured to convert analog audio input into a digital audio signal. The audio module 170 may be configured to code and decode an audio signal. In some embodiments, the audio module 170 may be disposed in the processor 110, or some functional modules in the audio module 170 are disposed in the processor 110.

The speaker 170A, also referred to as a "loudspeaker", is configured to convert an audio electrical signal into a sound signal. The electronic device 100 may be configured to listen to music or answer a hands-free call by using the speaker 170A.

The receiver 170B, also referred to as an "earpiece", is configured to convert an audio electrical signal into a sound signal. When a call is answered or voice information is received through the electronic device 100, the receiver 170B may be put close to a human ear to listen to a voice.

The microphone 170C, also referred to as a "mike" or a "microphone", is configured to convert a sound signal into an electrical signal. When making a call or sending a voice message, a user may make a sound near the microphone 170C through the mouth of the user, to input a sound signal to the microphone 170C. At least one microphone 170C may be disposed in the electronic device 100. In some other embodiments, two microphones 170C may be disposed in the electronic device 100, to collect a sound signal and implement a noise reduction function. In some other embodiments, three, four, or more microphones 170C may alternatively be disposed in the electronic device 100, to collect a sound signal, implement noise reduction, and identify a sound source, so as to implement a directional recording function and the like.

The headset jack 170D is configured to connect to a wired headset. The headset jack 170D may be the USB interface 130, or may be a 3.5 mm open mobile electronic device platform (open mobile terminal platform, OMTP) standard interface or a cellular telecommunications industry association of the USA (cellular telecommunications industry association of the USA, CTIA) standard interface.

The pressure sensor 180A is configured to sense a pressure signal, and can convert the pressure signal into an electrical signal. In some embodiments, the pressure sensor 180A may be disposed on the display 194. There are a plurality of types of pressure sensors 180A, such as a resistive pressure sensor, an inductive pressure sensor, and a capacitive pressure sensor. The capacitive pressure sensor may include at least two parallel plates made of conductive materials. When a force is applied to the pressure sensor 180A, capacitance between electrodes changes. The electronic device 100 determines pressure intensity based on the change in the capacitance. When a touch operation is performed on the display 194, the electronic device 100 detects intensity of the touch operation through the pressure sensor 180A. The electronic device 100 may calculate a touch location based on a detection signal of the pressure sensor 180A. In some embodiments, touch operations that are performed in a same touch position but have different touch operation intensity may correspond to different operation instructions. For example, when a touch operation whose touch operation intensity is less than a first pressure threshold is performed on an SMS message application icon, an instruction for viewing an SMS message is performed. When a touch operation whose touch operation intensity is greater than or equal to the first pressure threshold is performed on the SMS message application icon, an instruction for creating a new SMS message is performed.

The gyroscope sensor 180B may be configured to determine a moving posture of the electronic device 100. In some embodiments, an angular velocity of the electronic device 100 around three axes (namely, axes x, y, and z) may be determined through the gyroscope sensor 180B. The gyroscope sensor 180B may be configured to implement image stabilization during photographing. For example, when the shutter is pressed, the gyroscope sensor 180B detects an angle at which the electronic device 100 jitters, calculates, based on the angle, a distance for which a lens module needs to compensate, and allows the lens to cancel the jitter of the electronic device 100 through reverse motion, to implement image stabilization. The gyroscope sensor 180B may also be used in a navigation scenario and a somatic game scenario.

The barometric pressure sensor 180C is configured to measure barometric pressure. In some embodiments, the electronic device 100 calculates an altitude through the barometric pressure measured by the barometric pressure sensor 180C, to assist in positioning and navigation.

The magnetic sensor 180D includes a Hall sensor. The electronic device 100 may detect opening and closing of a flip cover leather case by using the magnetic sensor 180D. In some embodiments, when the electronic device 100 is a clamshell phone, the electronic device 100 may detect opening and closing of a flip cover based on the magnetic sensor 180D. Further, a feature such as automatic unlocking upon opening of the flip cover is set based on a detected opening or closing state of the leather case or a detected opening or closing state of the flip cover.

The acceleration sensor 180E may detect accelerations in various directions (usually on three axes) of the electronic device 100. When the electronic device 100 is still, a magnitude and a direction of gravity may be detected. The acceleration sensor 180E may be further configured to recognize a posture of the electronic device, and is used in screen switching between a landscape mode and a portrait mode, a pedometer, or another application.

The distance sensor 180F is configured to measure a distance. The electronic device 100 may measure a distance in an infrared manner or a laser manner. In some embodiments, in a photographing scenario, the electronic device 100 may measure a distance through the distance sensor 180F to implement quick focusing.

The optical proximity sensor 180G may include, for example, a light emitting diode (LED) and an optical detector, for example, a photodiode. The light-emitting diode may be an infrared light-emitting diode. The electronic device 100 emits infrared light by using the light-emitting diode. The electronic device 100 detects infrared reflected light from a nearby object through the photodiode. When sufficient reflected light is detected, it may be determined that there is an object near the electronic device 100. When insufficient reflected light is detected, the electronic device 100 may determine that there is no object near the electronic device 100. The electronic device 100 may detect, by using the optical proximity sensor 180G, that a user holds the electronic device 100 close to an ear for a call, to automatically perform screen-off for power saving. The optical proximity sensor 180G may also be used in a leather case mode or a pocket mode to automatically unlock or lock the screen.

The ambient light sensor 180L is configured to sense ambient light brightness. The electronic device 100 may adaptively adjust brightness of the display 194 based on the sensed ambient light brightness. The ambient light sensor 180L may also be configured to automatically adjust a white balance during photographing. The ambient light sensor 180L may also cooperate with the optical proximity sensor 180G to detect whether the electronic device 100 is in a pocket, to avoid an accidental touch.

The fingerprint sensor 180H is configured to collect a fingerprint. The electronic device 100 may use a feature of the collected fingerprint to implement fingerprint-based unlocking, application lock access, fingerprint-based shooting, fingerprint-based call answering, and the like.

The temperature sensor 180J is configured to detect a temperature. In some embodiments, the electronic device 100 executes a temperature processing policy through the temperature detected by the temperature sensor 180J. For example, when the temperature reported by the temperature sensor 180J exceeds a threshold, the electronic device 100 lowers performance of a processor nearby the temperature sensor 180J, to reduce power consumption for thermal protection. In some other embodiments, when the temperature is less than another threshold, the electronic device 100 heats the battery 142 to prevent the electronic device 100 from being abnormally powered off because of a low temperature. In some other embodiments, when the temperature is less than still another threshold, the electronic device 100 boosts an output voltage of the battery 142, to prevent abnormal power-off caused by a low temperature.

The touch sensor 180K is also referred to as a "touch panel". The touch sensor 180K may be disposed on the display 194, and the touch sensor 180K and the display 194 constitute a touchscreen, which is also referred to as a "touchscreen". The touch sensor 180K is configured to detect a touch operation performed on or near the touch sensor 180K. The touch sensor may transfer the detected touch operation to the application processor to determine a type of the touch event. A visual output related to the touch operation may be provided on the display 194. In some other embodiments, the touch sensor 180K may alternatively be disposed on a surface of the electronic device 100 at a position different from that of the display 194.

The bone conduction sensor 180M may obtain a vibration signal. In some embodiments, the bone conduction sensor 180M may obtain a vibration signal of a vibration bone of a human vocal-cord part. The bone conduction sensor 180M may also be in contact with a human pulse, to receive a blood pressure beating signal. In some embodiments, the bone conduction sensor 180M may also be disposed in the headset, to obtain a bone conduction headset. The audio module 170 may obtain a voice signal through parsing based on the vibration signal that is of the vibration bone of the vocal-cord part and that is obtained by the bone conduction sensor 180M, to implement a voice function. The application processor may parse heart rate information based on the blood pressure beating signal obtained by the bone conduction sensor 180M, to implement a heart rate detection function.

The button 190 includes a power button, a volume button, and the like. The button 190 may be a mechanical button, or may be a touch button. The electronic device 100 may receive a key input, and generate a key signal input related to a user setting and function control of the electronic device 100.

The motor 191 may generate a vibration prompt. The motor 191 may be configured to provide an incoming call vibration prompt or a touch vibration feedback. For example, touch operations performed on different applications (for example, shooting and audio playing) may correspond to different vibration feedback effects. The motor 191 may also correspond to different vibration feedback effects for touch operations performed on different areas of the display 194. Different application scenarios (for example, a time reminder, information receiving, an alarm clock, and a game) may also correspond to different vibration feedback effects. A touch vibration feedback effect may be further customized.

The indicator 192 may be an indicator light, and may be configured to indicate a charging status and a power change, or may be configured to indicate a message, a missed call, a notification, and the like.

The SIM card interface 195 is configured to connect to a SIM card. The SIM card may be inserted into the SIM card interface 195 or removed from the SIM card interface 195, to implement contact with or separation from the electronic device 100. The electronic device 100 may support one or N SIM card interfaces, where N is a positive integer greater than 1. The SIM card interface 195 can support a nano-SIM card, a micro-SIM card, a SIM card, and the like. A plurality of cards may be simultaneously inserted into a same SIM card interface 195. The plurality of cards may be of a same type or of different types. The SIM card interface 195 is compatible to different types of SIM cards. The SIM card interface 195 is also compatible to an external storage card. The electronic device 100 interacts with a network through the SIM card, to implement functions such as conversation and data communication. In some embodiments, the electronic device 100 uses an eSIM, that is, an embedded SIM card. The eSIM card may be embedded into the electronic device 100, and cannot be separated from the electronic device 100.

A software system of the electronic device 100 may use a layered architecture, an event-driven architecture, a microkernel architecture, a micro service architecture, or a cloud architecture. In embodiments of this application, an Android system with a layered architecture is used as an example to describe a software structure of the electronic device 100.

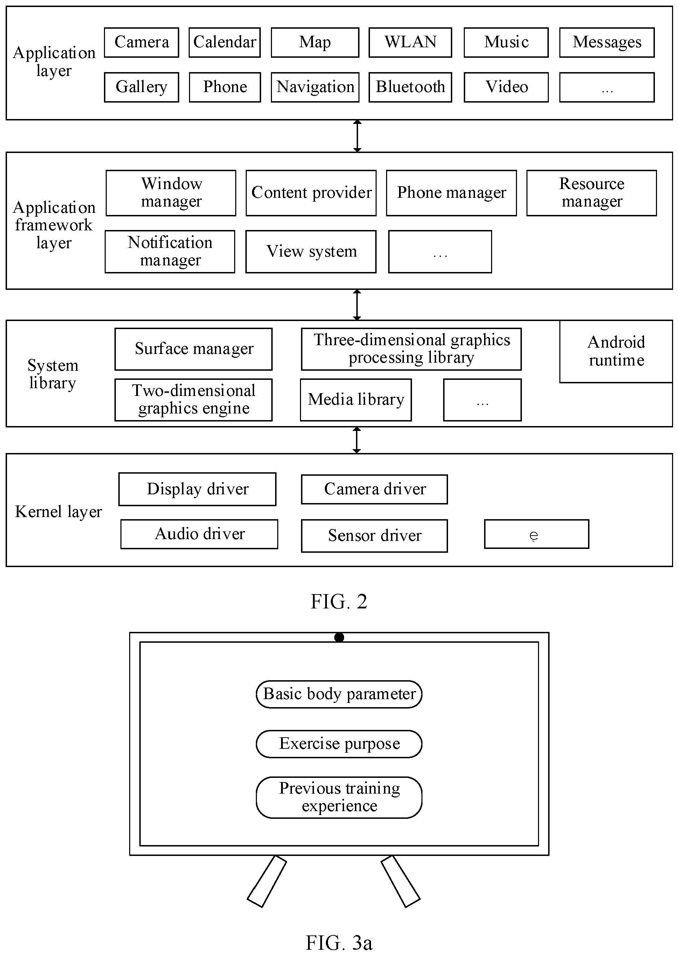

FIG. 2 is a block diagram of a software structure of an electronic device 100 according to an embodiment of this application.

In a layered architecture, software is divided into several layers, and each layer has a clear role and task. The layers communicate with each other through a software interface. In some embodiments, the Android system is divided into four layers: an application layer, an application framework layer, an Android runtime (Android runtime) and system library, and a kernel layer from top to bottom.

The application layer may include a series of application packages.

As shown in FIG. 2, the application packages may include applications such as Camera, Gallery, Calendar, Phone, Map, Navigation, WLAN, Bluetooth, Music, Videos, and Messages.

The application framework layer provides an application programming interface (application programming interface, API) and a programming framework for an application at the application layer. The application framework layer includes some predefined functions.

As shown in FIG. 2, the application framework layer may include a window manager, a content provider, a view system, a phone manager, a resource manager, a notification manager, and the like.

A window manager is configured to manage a window program. The window manager may obtain a size of a display, determine whether there is a status bar, perform screen locking, take a screenshot, and the like.

The content provider is configured to store and obtain data, and enable the data to be accessed by an application. The data may include a video, an image, audio, calls that are made and answered, a browsing history and bookmarks, an address book, and the like.

The view system includes visual controls such as a control for displaying a text and a control for displaying a picture. The view system may be used to construct an application. A display interface may include one or more views. For example, a display interface including an SMS message notification icon may include a text display view and a picture display view.

The phone manager is configured to provide a communication function for the electronic device 100, for example, management of a call status (including answering, declining, or the like).

The resource manager provides various resources for an application such as a localized character string, an icon, a picture, a layout file, and a video file.

The notification manager enables an application to display notification information in a status bar, and may be configured to convey a notification message. The notification manager may automatically disappear after a short pause without requiring a user interaction. For example, the notification manager is configured to notify download completion, provide a message notification, and the like. The notification manager may alternatively be a notification that appears in a top status bar of the system in a form of a graph or a scroll bar text, for example, a notification of an application running on the background or a notification that appears on a screen in a form of a dialog window. For example, text information is prompted in the status bar, an alert sound is played, the electronic device vibrates, or the indicator light blinks.

The Android runtime includes a kernel library and a virtual machine. The Android runtime is responsible for scheduling and management of the Android system.

The kernel library includes two parts: a function that needs to be called in Java language and a kernel library of Android.

The application layer and the application framework layer run on the virtual machine. The virtual machine executes Java files of the application layer and the application framework layer as binary files. The virtual machine is configured to implement functions such as object lifecycle management, stack management, thread management, security and abnormality management, and garbage collection.

The system library may include a plurality of function modules such as for example, a surface manager (surface manager), a media library (Media Library), a three-dimensional graphics processing library (for example, OpenGL ES), and a 2D graphics engine (for example, SGL).

The surface manager is configured to manage a display subsystem, and provide fusion of 2D and 3D layers for a plurality of applications.

The media library supports playback and recording in a plurality of commonly used audio and video formats, static image files, and the like. The media library may support a plurality of audio and video coding formats such as MPEG4, H.264, MP3, AAC, AMR, JPG, and PNG.

The three-dimensional graphics processing library is configured to implement three-dimensional graphics drawing, image rendering, compositing, layer processing, and the like.

The 2D graphics engine is a drawing engine for 2D drawing.

The kernel layer is a layer between hardware and software. The kernel layer includes at least a display driver, a camera driver, an audio driver, and a sensor driver.

The following describes an example of a working process of software and hardware of the electronic device 100 with reference to a capture photographing scenario.

When the touch sensor 180K receives a touch operation, a corresponding hardware interruption is sent to the kernel layer. The kernel layer processes the touch operation into an original input event (including information such as touch coordinates and a time stamp of the touch operation). The original input event is stored at the kernel layer. The application framework layer obtains the original input event from the kernel layer, and identifies a control corresponding to the input event. For example, the touch operation is a single-tap operation and a control corresponding to the single-tap operation is a control of a camera application icon. A camera application invokes an interface at the application framework layer, to start the camera application. Then, the camera driver is started by invoking the kernel layer, and a static image or a video is captured by using the camera 193.

Specifically, when executing the executable program code in the internal memory 121, the processor 110 of the electronic device 100 may implement the steps performed in the adaptive action evaluation method provided in embodiments of this application. The electronic device 100 may be specifically a television.

In modem society, pace of life of people is getting faster and faster, and there are more or fewer physical health problems. How to maintain a good physical condition under fast-paced work becomes an urgent problem to be resolved. Currently, using a television (an intelligent television or an intelligent screen) to perform intelligent exercise guidance is a good home exercise solution. However, exercise capabilities and body coordination of different individuals vary greatly. It is obviously inaccurate to measure an action completion status by using a single evaluation standard for all users, and not only cannot effectively guide the user to complete exercise, but even may cause body damage to the user in some action training processes.

Currently, most exercise applications on the television do not support action guidance. Most exercise applications only function as video players. Only a few applications that implement action recognition implement simple statistics collection. Further, even if the exercise applications support the intelligent action guidance, action evaluation feedback is provided only based on a determined measurement standard, and targeted training cannot be performed on different users, to achieve an optimal effect of individual training.

In view of this, this application provides an adaptive action evaluation method, which can provide a differentiated service for the user, so that the user can perform effective physical exercise and improve physical quality of the user.

In an embodiment, the user uses a television (in embodiments of this application, the electronic device 100 may specifically refer to the television, and the television may also be referred to as an intelligent screen, and has a function such as intelligent interaction with the user, and is not limited to a conventional television used to watch multimedia information such as a video) to perform exercise in an account registration phase, and the first evaluation threshold may be set based on a basic body parameter, an exercise purpose, a previous training experience, or the like of the user. The first evaluation threshold is used to provide an evaluation standard, and may reflect information such as physical quality and an interest direction of the user participating in exercise on the whole. The first evaluation threshold may be specifically a value in a range of [0, 1]. For example, when the basic body parameter of the user is excellent (for example, a body mass index (Body Mass Index, BMI) meets a standard, and only the BMI is used as an evaluation parameter), a previous training experience is rich, and an exercise purpose is to reduce fat by performing exercise, weights corresponding to the three conditions may be all 1/3, and coefficients are all 1. After weighting is performed on the three conditions, the first evaluation threshold may be obtained to be 100%. The first evaluation threshold is 100%, which indicates that information input by the user, such as the basic body parameter, the exercise purpose, and the previous training experience in the account registration phase may be used to learn that physical quality of the user is relatively good, and the user frequently performs exercise and expects to obtain a relatively good fat reduction result by performing exercise this time. Therefore, the evaluation standard of the user is set to be high. It may be understood that under the high evaluation standard, the user needs to perform more standard actions to continue actions in the subsequent phases, and the user performs more targeted (such as fat reduction) intelligent exercise. Relatively, when the basic body parameter of the user is average, the previous training experience is little, and the exercise purpose is entertainment only, weights corresponding to the three conditions may be all 1/3, coefficients are all 0.6, and the first evaluation threshold is 60% after weighting is performed on the three conditions. The first evaluation threshold is 60%, which indicates that the user is in the account registration phase. Based on information input by the user such as the basic body parameter, the exercise purpose, and the previous training experience, it may be learnt that the physical quality of the user is average, the user does not perform exercise frequently, and the exercise purpose is entertainment only in exercise performed this time. Therefore, the evaluation standard of the user is set to be relatively low. It may be understood that when the user performs exercise under a relatively low evaluation standard, the user relatively easily completes the current action and enters an action in a next phase. In addition, under the evaluation standard, the action completion status that is fed back is mostly "perfect" or "great". It may be understood that in a scenario of the relatively low evaluation standard, the intelligent exercise on the television is mainly used to encourage the user to perform exercise, and a requirement for a completion degree of the user action is not high.

It may be understood that the first evaluation threshold may distinguish physical conditions of different users, and set different evaluation standards for different users. The evaluation standard refers to a standard that is set and that is determined and suitable for different users after the basic information parameter of the user is analyzed. For example, if a result obtained after the basic information of the user is analyzed is 100%, it indicates that a user action may be evaluated based on a highest standard. If a result obtained after the basic information of the user is analyzed is 80%, it indicates that a current physical status of the user is not suitable for performing evaluation based on the highest standard, and evaluation is performed on the user action based on 80% of the highest standard. Further, in a subsequent exercise process, the first evaluation threshold may be adjusted based on an actual action completion status of the user.

Further, in a user account registration phase, in addition to a questionnaire, the user may be further required to complete a set of physical fitness evaluation actions (such as functional movement screen (FMS), or the like), to evaluate an exercise capability of the user, and complete setting of the first evaluation threshold of the user. Specifically, for example, FMS evaluation, the FMS evaluation may be used to detect capabilities such as overall action control stability, a body balance capability, flexibility, and ontology feeling of the user, and an overall physical condition of the user may be easily recognized through the FMS evaluation. In an embodiment, accuracy of setting the first evaluation threshold by using the physical fitness evaluation action may be higher than accuracy of setting a threshold in a manner in which the user inputs the information such as the basic body parameter, the exercise purpose, and the previous training experience.

In an embodiment, the information of the user such as the basic body parameter, the exercise purpose, and the previous training experience may be entered through television interface interaction. Specifically, the information that needs to be entered in advance may be instructed based on a specific sequence, and the information is entered through interface interaction.

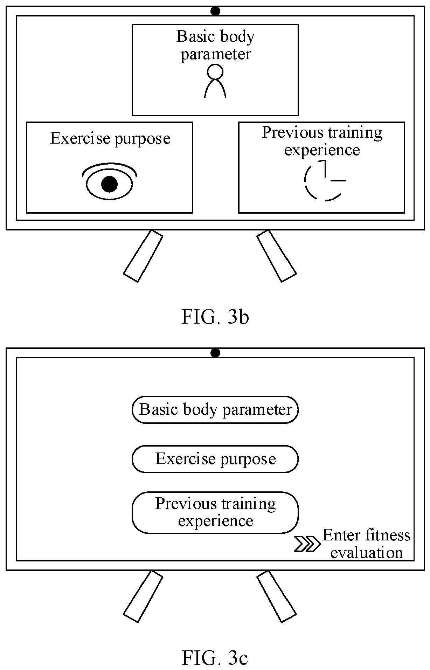

FIG. 3a and FIG. 3b are schematic diagrams of implementing information entry through television interface interaction. It may be seen from FIG. 3a and FIG. 3b that, a plurality of entry controls (such as buttons and pictures) for entering information are displayed on the television interface. After using an interaction manner such as tapping, gesture control, or the like, the user may enter a corresponding information entry interface based on a selected entry control. Specifically, in FIG. 3a, a button-type entry control is used. The user may select a button option such as the basic body parameter by performing an operation such as tapping, to enter an information entry interface of the basic body parameter. In FIG. 3b, a tapable picture-type entry control is used. The user may select, in an operation manner such as tapping, a picture of the previous training experience, to enter an information entry interface of the previous training experience. It may be understood that an implementation of the entry control is not specifically limited, provided that the user may implement an objective of entering information through the television interface interaction.

FIG. 3c is a schematic diagram of implementing physical fitness evaluation through television interface interaction. It may be seen from FIG. 3c that, in addition to a manner of entering information such as the basic body parameter, the user may further select the physical fitness evaluation through the television interface. Specifically, the user may select a physical fitness evaluation entry by performing an operation such as tapping, to perform physical fitness evaluation.

In an embodiment, the basic body parameters of the user may include parameters such as a BMI, a body fat percentage, completion time for running 1,000 kilometers, vital capacity, grip force, bench press force, and foot thrust force. The basic body parameters reflect the overall basic physical condition of the user, and have a reference significance for setting the first evaluation threshold. Further, the setting of the first evaluation threshold may be dynamically adjusted based on an action (one or more groups) selected by the user or recommended by a system. If the user selects fat reduction exercise, the BMI, the body fat percentage, or the like may be used as an item with a relatively high weight, to more accurately obtain evaluation (such as excellent, good, and average) of the basic body parameter of the user. It may be understood that in different action scenarios, a value of the first evaluation threshold is dynamically adjusted instead of being fixed.

In an embodiment, an exercise action recommended by the system may be determined based on the exercise purpose of the user. When the exercise purpose of the user is fat reduction by performing exercise, the system mainly recommends an action related to fat reduction. Further, in addition to a specific exercise direction, the exercise purpose may further include an exercise expectation of the user. For example, the exercise expectation of the user may be a full and effective exercise effect, or may be an exercise expectation for entertainment and exercise only. It may be understood that the exercise expectation may also be used as a weight item in the exercise expectation to adjust the first evaluation threshold.

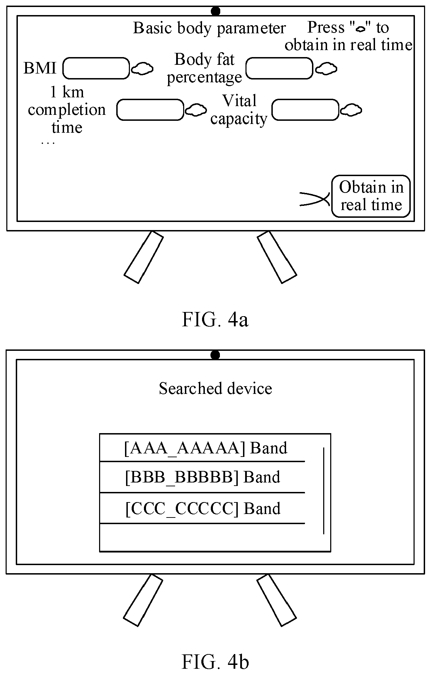

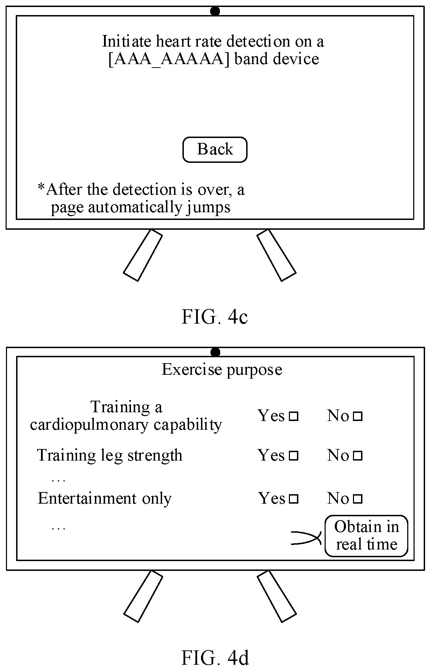

FIG. 4a is a schematic diagram of entering a basic body parameter of a user through a television interface. It may be seen from FIG. 4a that the user may fill in the basic body parameter on the television interface in a filling manner. It may be understood that the user does not necessarily know all basic body parameters of the user. Therefore, related basic body information may be automatically filled in in a real-time entering manner. Specifically, as shown in FIG. 4a, the user may trigger, by selecting a "real-time obtaining" button, a connection operation of a device related to the basic body parameter. Specifically, if the user wants to fill in the heart rate of the user, when detecting a requirement of the user for selecting to obtain the heart rate in real time, the television searches for an electronic device that can measure the heart rate, such as a smart band (with a heart rate measurement function), and connects to the electronic device. FIG. 4b and FIG. 4ab are schematic diagrams of a communication connection between a television and a smart band. It may be seen from FIG. 4b that when detecting a smart band on which the heart rate may be measured, the television may display, on the television interface, the smart band that may be connected. As shown in FIG. 4c, after the user selects the band that the user wears, the user is prompted, on the television interface, to perform a heart rate measurement operation by using the connected smart band. After the user measures the heart rate through the smart band, the smart band sends the heart rate to the connected television. After receiving the heart rate, the television jumps to the television interface on which the basic body parameters are entered, and automatically fills the heart rate (an average value may be used) into a heart rate item on the television interface on which the basic body parameters are entered.

Further, as shown in FIG. 4a, the user may further enter a function of the basic body parameter through an associated account. Specifically, in FIG. 4a, the user may obtain the basic body parameters of the user by selecting a button of "obtaining through association" through a cloud that has an associated account relationship with the television, where the basic body parameters may be uploaded to the cloud by another electronic device that has an associated account relationship with the television. For example, a smart band that has an associated account relationship with the television uploads the heart rate of the user to the cloud of the associated account, or uploads the body fat percentage of the user to the cloud of the associated account through a body fat scale. Further, the obtaining of the basic body parameters through association does not necessarily need to be implemented through the cloud, and may further be implemented in a manner of directly communicating with another electronic device that has an associated account relationship with the television. For example, the television initiates a service of obtaining the basic body parameters. After receiving the service, the electronic device of the associated account may send the basic body parameters related to the user to the television, to complete quick entry of the basic body parameters.

FIG. 4d is a schematic diagram of entering an exercise purpose of a user through a television interface. It may be seen from FIG. 4d that the exercise purpose of the user may be specifically a specific purpose such as training a cardiopulmonary capability, waist strength, leg strength, or the like. For the exercise purpose with a clear objective, it may be considered that the user has a rewarding exercise expectation for exercise. In this case, when the first evaluation threshold is set, a coefficient of the first evaluation threshold may be set to 1 (100%). Further, when the exercise purpose of the user is specifically training the cardiopulmonary capability, the television pushes exercise related to training of the cardiopulmonary capability, so that the user may complete training more purposefully. Further, as shown in FIG. 4d, the user may obtain the exercise purpose of the user through selecting a button of "obtaining through associated" and through the cloud or the electronic device that has the associated account relationship with the television. It may be understood that the user may already include information about the exercise purpose on the electronic device that has the associated account relationship with the television. For example, information related to the user may be collected on the electronic device of the associated account, so that an ongoing exercise plan and a specific exercise habit of the user may be obtained. For example, information related to the user is obtained through a mobile phone of the user. In this embodiment, the user may quickly enter the exercise purpose of the user into the television through the electronic device and the cloud that have the associated account relationship.

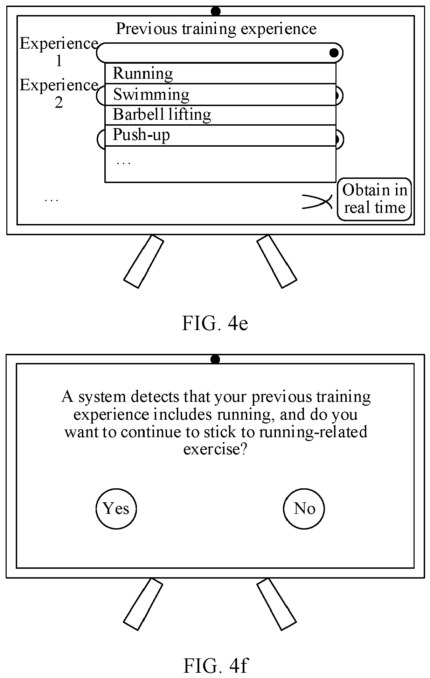

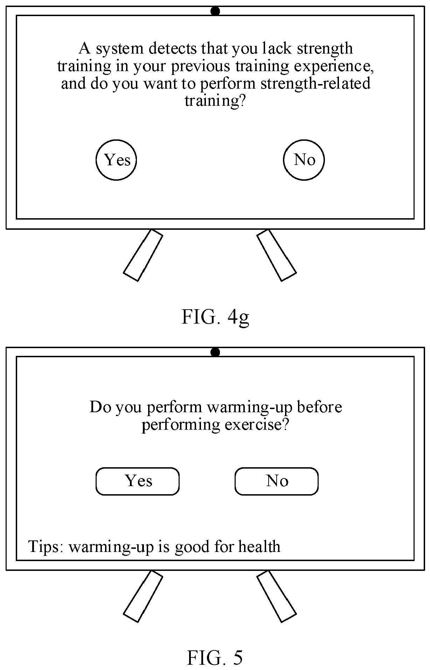

FIG. 4e is a schematic diagram of entering a previous training experience of a user through a television interface. It may be seen from FIG. 4e that, there is an input interface on the television interface, and the user may enter the previous training experience, such as running, swimming, push-up, and barbell lifting, in a manner of filling in text on the interface or pulling down an option box. Further, personal evaluations of the previous training experience may be selectively recorded, for example, running and swimming are excellent, and push-up and barbell lifting are average. The personal evaluations of the previous training experience may be used to calculate or adjust the first evaluation threshold. Further, as shown in FIG. 4e, the user may obtain the previous training experience of the user by selecting a button of "associated obtaining" and through the cloud or the electronic device that has the associated account relationship with the television. Specifically, for example, it may be determined, through running data of the user in the smart band, that the user frequently runs. In an embodiment, as shown in FIG. 4f, the television may prompt, on the interface, the user whether to continue to perform running-related training, or select another training item of a body part that is difficult to exercise during running. The user may select a preferred training item through the interface. In an embodiment, as shown in FIG. 4g, the television may further obtain, based on analysis, that the user lacks strength training based on the personal evaluations: running and swimming are excellent, and push-up and barbell lifting are average, and may prompt, on the television interface based on the personal evaluations, that the user lacks strength training and whether to perform exercise in terms of strength. It may be understood that when the user records the previous training experience of the user, not only the first evaluation threshold may be set, but also a proper suggestion may be provided for an exercise direction of the user, so that the exercise of the user is more targeted.

It may be understood that after information of the user such as the basic body parameter, the exercise purpose, and the previous training experience is obtained, the first evaluation threshold may be obtained in a manner of performing weighted calculation. The first evaluation threshold may be considered as a basic evaluation standard for the user. Specifically, the basic information parameter is classified based on a preset parameter classification standard, for example, classified into a plurality of types, such as the basic body parameter, the exercise purpose, and the previous training experience. Then, a first weight is assigned to the basic information parameters of a same category, and an evaluation result of the category is obtained through performing weighted calculation based on the basic information parameters of the same category. For example, the basic body parameter may be divided into a plurality of parameters, and a first weight is assigned to parameters of the same category, and an evaluation result (which may be a specific value) of a type of the basic body parameter may be obtained through performing weighted calculation. Finally, a second weight is assigned to basic information parameters of different categories, and the first evaluation threshold is obtained through performing weighting based on evaluation results of different categories. For example, after evaluation results of the basic body parameter, the exercise purpose, and the previous training experience are respectively obtained, the second weight is assigned to the basic information parameters of the different categories, and the first evaluation threshold may be obtained by performing weighted calculation.

Further, in addition to setting the first evaluation threshold based on the basic body parameter, the exercise purpose, and the previous training experience of the user, one more warm-up phase may be added. The second evaluation threshold is obtained based on the first evaluation threshold and parameter data obtained in the warm-up phase. Specifically, during a phase in which the user performs warming-up, the television continuously collects user image information, and detects a skeleton node of the user based on the image information. In a process of performing a stretching action by the user, the television calculates parameters such as a key angle and duration of the action completed by the user by using the detected skeleton node. These parameters may be preset based on different actions, for example, an inclination angle of a head stretching leftward or rightward, an inclination angle of a waist stretching leftward or rightward, duration of plank, and duration of some fixed actions. Further, historical related data of warming-up may also be used as parameters collected in the warm-up phase.

In an embodiment, before entering formal intelligent exercise, the user may select whether to perform warming-up on the television interface. Specifically, FIG. 5 is a schematic diagram of entering a warm-up scenario through a television interface. It may be seen from FIG. 5 that the television interface prompts the user whether to perform warming-up, and indicates that a warm-up process helps perform an action more standard and effective. The user may select to confirm a warm-up action before entering the warm-up scenario to perform the intelligent exercise. A specific warm-up action is associated with an action in the intelligent exercise phase to some extent. For example, if an action of a head training type is performed in the intelligent exercise phase, the warm-up action is also correspondingly related to head training warming-up. It may be understood that the warm-up phase is optional for the user, and the evaluation parameter obtained in the warm-up phase may be converted into a warm-up completion degree represented by a value, so that an evaluation standard of the user action may be dynamically adjusted more accurately, to obtain a more proper second evaluation threshold.

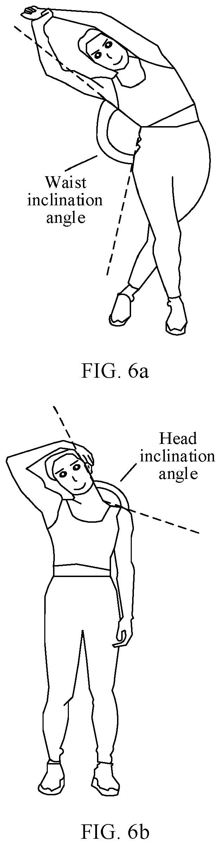

FIG. 6a and FIG. 6b are schematic diagrams of guiding a user to perform a warm-up action. FIG. 6a and FIG. 6b respectively correspond to two different types of warm-up actions: a head inclination angle and a waist inclination angle. It may be understood that for different types of warm-up actions, parameters such as a key angle or duration corresponding to the different types of warm-up actions are different. The television may require, based on a specific sequence, the user to perform a warm-up action that is the same as the warm-up action displayed on the interface of the television, and record the parameters in real time such as the key angle and the duration when the action is completed, to determine a current physical condition of the user based on a warm-up result of the user, and flexibly adjust an evaluation standard of a training process.

It may be understood that when the user performs warming-up, a camera of the television continuously monitors an action of the user, and determines a specific value of the parameter based on an image/video recognition result. For example, as shown in FIG. 6a, when the user performs waist stretching warm-up exercise, recognition calculation is performed on an angle shown in FIG. 6a based on the skeleton node of the user, to obtain a minimum angle that may be reached by the user, for example, 110 degrees. Similarly, as shown in FIG. 6b, when the user performs head stretching warm-up exercise, recognition calculation is performed on an angle shown in FIG. 6b based on the skeleton node of the user, to obtain a maximum angle that may be reached by the user, for example, 130 degrees. Further, in addition to calculation of parameters such as the key angle in the phase in which the user performs warming-up, parameters such as the duration and a jump height may also be calculated. For example, when the warming-up is push-up, a parameter that needs to be specially recorded in the warming-up is duration of push-up performed by the user.

In an embodiment, in addition to a photographing device provided by the television, functions such as angle recognition and calculation may be implemented in combination with other photographing devices. It may be understood that generally, only a 2D image may be obtained by using the photographing device provided by the television, and recognition accuracy of a parameter that has a high requirement for spatial observation such as an angle is average. In embodiments of this application, a parameter such as an angle may be detected from different observation angles in combination with other photographing devices.

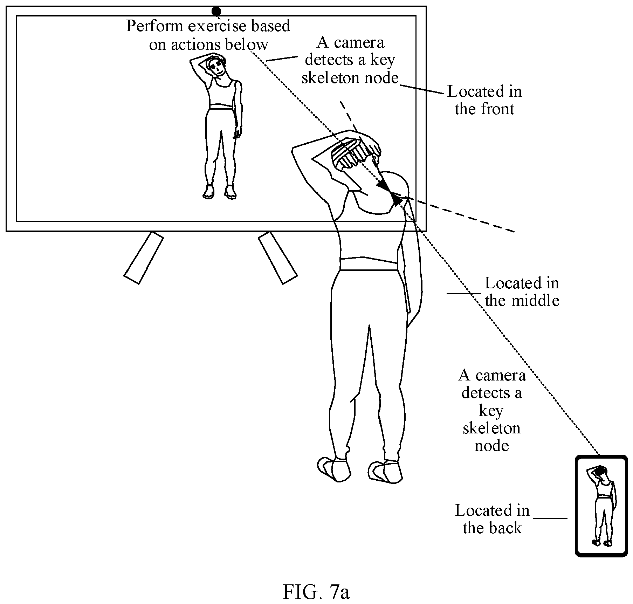

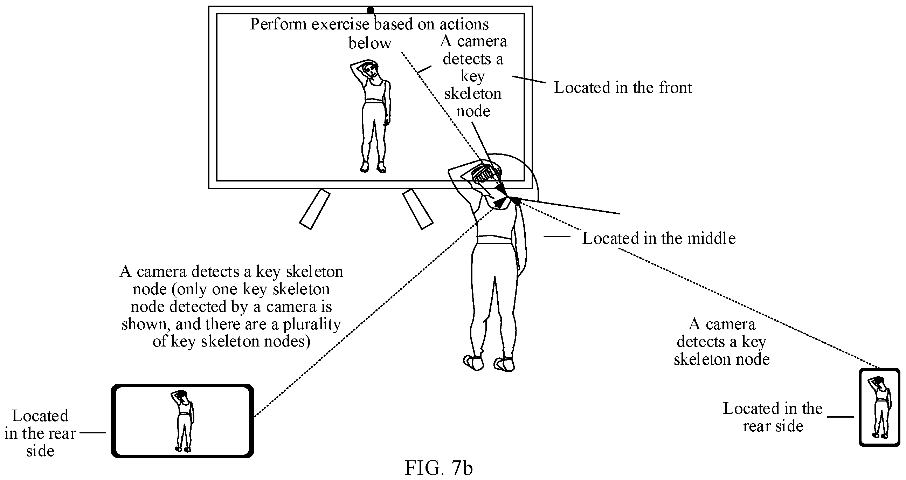

FIG. 7a is a schematic diagram of recognizing an inclination angle by using a plurality of photographing devices when a user performs a head stretching warm-up action. It may be seen from FIG. 7a that behind the user, the user may be shoot by using a camera of a mobile device. In combination with shooting performed on the user by a front side of the television, multi-dimensional shooting may be performed on the user from the front side and the back side of the user, to obtain more angle information. FIG. 7b is another schematic diagram of recognizing an inclination angle by using a plurality of photographing devices when a user performs a head stretching warm-up action. It may be seen from FIG. 7b, three photographing devices may be used to simultaneously collect the inclination angle of the user, to obtain more angle information. Specifically, the camera of the television is responsible for shooting the user from the front side. The mobile device (such as a tablet computer) on a lower left side in FIG. 7b shoots the user from a left side behind the user. The mobile device (such as a mobile phone) on a lower right side in FIG. 7b shoots the user from a right side behind the user. A user image shot by the television and a user image shot by the mobile device on the lower left side in FIG. 7b may be used to obtain user images at three different angles. The head inclination angle of the user is calculated more accurately through the three user images. In addition to the embodiment shown in FIG. 7a or FIG. 7b, another feasible multi-camera device solution (for example, a solution of four cameras) should also be included in the solution of this application. For example, in an electronic device having a plurality of cameras, for example, in a case that one television includes a plurality of cameras, a plurality of different images that are obtained by shooting by the plurality of cameras of the television may also help calculate the head inclination angle of the user more accurately. In embodiments of this application, angle information collected by using the solution shown in FIG. 7a or FIG. 7b may be calculated through a preset algorithm (for example, a neural network algorithm) to obtain an optimal head inclination angle of the user, which is more accurate than the head inclination angle of the user calculated by using a 2D image collected by the photographing device of the television separately. It should be noted that the multi-camera device solution used in embodiments of this application is not limited to angle recognition, and may further be 3D recognition of other action-related parameters (such as a jump height and a jump distance). Further, in addition to applying the multi-camera device solution in a warm-up process, related parameters for completing an action may be recognized and calculated in real time in a process in which the user performs exercise, so that accuracy of evaluation may be improved.

Further, when a key evaluation parameter of a current action of the user is obtained, the following steps may be specifically included:
(1) determining a type of the key evaluation parameter of the current action;
(2) starting a corresponding monitoring module based on the type of the key evaluation parameter of the current action; and
(3) obtaining the key evaluation parameter of the current action by using the monitoring module.

The monitoring module includes a photographing module (camera), a timing module, a length, a distance measurement module, an AI intelligence module, or the like. Specifically, when the type of the key evaluation parameter is the action angle, the photographing module is started, and the action angle of the current action is obtained by using the photographing module; and when the type of the key evaluation parameter is the duration, the photographing module and the timing module are started, and the duration of the current action is obtained by using the photographing module and the timing module. It may be understood that the television may include a plurality of monitoring modules such as the photographing module and the timing module. When different actions are monitored, a corresponding module is automatically invoked based on the type of the key evaluation parameter. It may be understood that the monitoring module may be configured inside the television, or may be provided by a peripheral device, which is not limited herein.

Further, parameters such as the key angle and the duration when the user action is completed may be used to feed back the parameters to an action evaluation module of the television, to obtain the second evaluation threshold through adjustment based on the first evaluation threshold. Classification results of different evaluations on the user may be displayed on the interface based on the second evaluation threshold. Specifically, if the first evaluation threshold is 100% (1), when completion quality of the warm-up action of the user is greater than 50% and less than 70% under the first evaluation threshold, the first evaluation threshold is adjusted based on a preset adjustment rule. For example, if the completion quality of the warm-up action is greater than 50% and less than 70%, the first evaluation threshold is decreased by 20%. In this case, the first evaluation threshold 100% is decreased to 80%, to obtain the second evaluation threshold of 80% that is temporarily adjusted based on the warm-up status. The second evaluation threshold may be considered as the first evaluation threshold adjusted based on the warm-up status. This is only to distinguish between fine-tuned values obtained after the warm-up and distinguish the second evaluation threshold and the first evaluation threshold in terms of appellation. When the completion quality of the warm-up action of the user is greater than 50% but less than 70%, the television interface prompts an entry level and reminds the user to stick to performing exercise. A level may be increased by simulating a standard action (as the level increases, the first evaluation threshold may be simultaneously increased). Similarly, when the completion quality of the warm-up action of the user is greater than 70% and less than 90%, the television interface prompts an average level. When the completion quality of the warm-up action of the user reaches or is greater than 90%, the interface prompts a professional level. Training of the user at different levels corresponds to different evaluation standards, and training requirements of different users may be met.

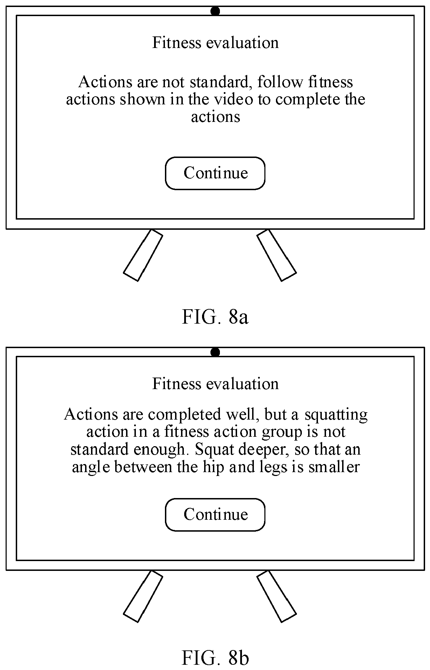

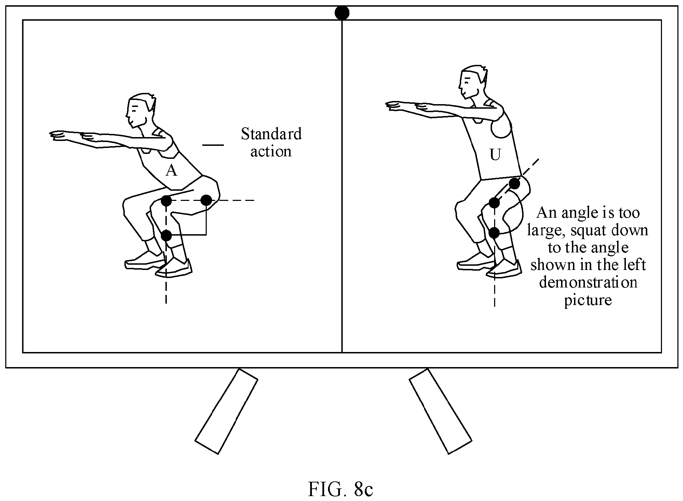

In an embodiment, correction reminding information of the action may be further displayed on the interface based on the (adjusted) first evaluation threshold. Specifically, FIG. 8a to FIG. 8c are schematic diagrams of action correction reminding information. When the completion quality of the user action is in a range of 50% to 70% at the adjusted first evaluation threshold, it may be seen from FIG. 8a that only an evaluation result (for example, excellent, good, and average) is provided on the television interface, and the user is reminded to perform action correction based on the action. When the completion quality of the user action is in a range of 70% to 90%, it may be seen from FIG. 8b that the television interface prompts the user which specific actions need to be corrected and a description of how to perform correction. When the completion quality of the user action is greater than 90%, it may be seen from FIG. 8c that a difference between the user action and a demonstration action is analyzed and displayed on the television interface, and how to correct the action is specifically described. In FIG. 8b and FIG. 8c, at least the photographing module of the television is invoked to detect the user action in real time, and correction action descriptions in FIG. 8b and FIG. 8c are output based on a preset determining set. It may be understood that a higher adjusted first evaluation threshold indicates a higher evaluation standard and a higher accuracy requirement for the user action. For completion quality of different actions, different action correction methods may be adopted. For example, when the completion quality of the warm-up action of the user is in a range of 50% to 70%, it indicates that an evaluation standard in this case is relatively low. When the user performs exercise, the user only needs to perform simulation based on the action to meet a requirement under the evaluation standard (at least 50 points under the current first evaluation threshold standard). In this case, the user is only reminded to perform normal training based on the action. When the completion quality of the warm-up action of the user is in a range of 70% to 90%, it indicates that the evaluation standard in this case is average. When the user performs exercise, actions performed by the user may be determined based on the collected image information, and the user may be reminded to seriously imitate a specific action. When the completion quality of the warm-up action of the user is greater than 90%, it indicates that the evaluation standard in this case is relatively high. When the user performs exercise, the user does not know how to correct the action even if the user knows which action is not good. In this case, based on the preset determining set, for example, when the action is squatting, and an angle of the squatting action of the user deviates too much, an image of the squatting action of the user may be compared with an image of a training action. After seeing analysis and comparison on the interface, the user may correct the action in a targeted manner until an optimal exercise effect is achieved.

It may be understood that if the user does not perform warming-up or does not have warm-up historical data before performing exercise, an action correction function may also be directly implemented based on the first evaluation threshold. Details are not described herein.

In an embodiment, in a process of performing exercise by the user, based on the real-time action completion evaluation of the user (for example, the action completion evaluation whose score is higher than 90 points is excellent, and another action completion evaluation is set in a similar manner), an evaluation standard of the action may be further adaptively iterated (that is, the second evaluation threshold is adjusted), and a user level is visually enhanced on the interface until a highest evaluation standard is reached, where the action completion evaluation is determined based on the key evaluation parameter summarized based on the exercise and the current first evaluation threshold. Specifically, when an average score of the first three times of training performed by the user is higher than 90 points (excellent), the user level may be correspondingly increased by one level, for example, from the average level to the professional level. Specifically, under an evaluation standard of the first evaluation threshold of 80%, when the average score of the first three times of training performed by the user is higher than 90 points (excellent), the first evaluation threshold may be increased by 10%. The first evaluation threshold changes to 90%. It may be understood that when the first evaluation threshold increases, it indicates that an upper limit of the score is also increased. The user needs to continue to increase the user level or the first evaluation threshold until the user level or the first evaluation threshold reaches a maximum value when the average score of the first three times of training performed by the user is still higher than 90 points (90 points whose first average threshold is 90% are different from 90 points whose first average threshold is 80%) under the increased first evaluation threshold. When the average score of the first three times of training performed by the user is lower than 70 points (average), the user level may be correspondingly decreased by one level, for example, decreased from the average level to the entry level, and the first evaluation threshold is decreased. When the user level is already the entry level (lowest level), the user level is not decreased.

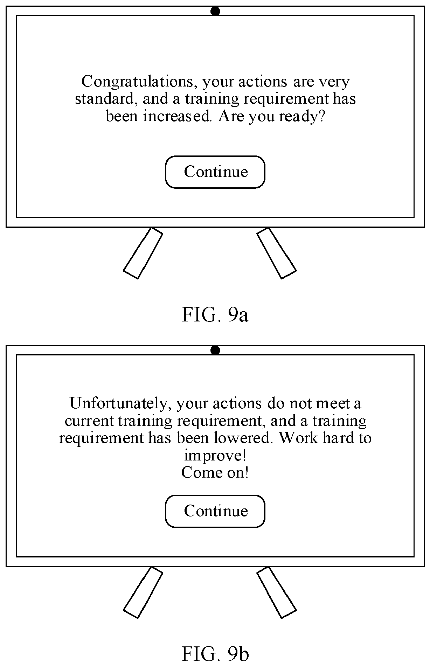

Specifically, FIG. 9a to FIG. 9b are schematic diagrams of interfaces of an adaptive and iterative action evaluation standard (a first evaluation threshold) based on a real-time action completion status of a user. As shown in FIG. 9a, when the average score of the first three times of training performed by the user is higher than 90 points, the television prompts, on an interface, that the evaluation standard of the user action has been upgraded, and prompts the user that a more standard action needs to be performed. When the average score of the first three times of training performed by the user is lower than 70 points, the television prompts, on the interface, that the evaluation standard of the user action has been degraded, and prompts the user to improve the evaluation standard from the relatively low standard.

In embodiments of this application, the user implements a function of adaptive action evaluation through intelligent interaction with the television interface. Specifically, the television first obtains the basic information parameter of the user through an interface displayed on the screen. The basic information parameter may be specifically divided into content such as a basic body parameter, an exercise purpose, and a previous training experience, and the basic information parameter may further be obtained through entering physical fitness evaluation. As shown in FIG. 3a and FIG. 3b, the user may complete entry of the basic information parameter in a selection manner such as tapping and selecting. Further, the basic information parameter may be manually input by the user, or the basic information parameter may be synchronously obtained by the user in real time through a cloud, an electronic device that has a same account, or the like, and may be automatically filled on the interface. When searching for the cloud and the electronic device that has the same account, as shown in FIG. 4b and FIG. 4c, the user may select a correct available electronic device through prompt information displayed on the television interface. After the basic information parameter of the user is entered, the user may be further prompted to perform warming-up through the interface, to preliminarily and dynamically adjust, based on the key evaluation parameter (a parameter used to evaluate a user action completion status, where there may be a plurality of key evaluation parameters, for example, a head inclination angle, a waist inclination angle, and a knee bending angle of the user) in the warm-up action, the first evaluation threshold set for the basic information parameter, which helps set a more proper evaluation standard based on physical conditions of different users. Finally, when the user performs exercise, the current first evaluation threshold may be adjusted in real time through the key evaluation parameter in the action obtained in real time, and the first evaluation threshold is increased or decreased based on the user action completion status. When the user action is performed in a relatively standard manner, a higher requirement is raised, or when the user action is performed in a non-standard manner, a requirement is appropriately reduced, to improve user participation positiveness. In the foregoing implementation process, the user may complete intelligent information interaction with the television through the television interface in an entire process. The television makes an intelligent response based on specific information (including image information shot by the photographing device) input by the user, provides differentiated exercise services for different users, and dynamically, flexibly, and properly adjusts evaluation standards.

In embodiments of this application, the first evaluation threshold is set by obtaining the basic information parameter of the user, so that a more proper evaluation standard may be set based on physical conditions of different users. When the user performs exercise, through obtaining the key evaluation parameter of the current user action, and the first evaluation threshold that is set, the action completion evaluation of the user is determined, to flexibly adjust the first evaluation threshold based on an actual completion status of the user action. In embodiments of this application, a differentiated exercise service can be provided for the user, so that the user can perform targeted physical exercise and effectively improve physical quality of the user.

This application further provides a computer storage medium. The computer-readable storage medium stores a computer program. When the computer program is executed by a processor, steps of the adaptive action evaluation method in the embodiments are implemented. To avoid repetition, this is not described herein again.

It should be clear that the described embodiments are merely some rather than all of embodiments of this application. All other embodiments obtained by a person of ordinary skill in the art based on embodiments of this application without creative efforts shall fall within the protection scope of this application.

It may be clearly understood by a person skilled in the art that, for convenient and brief description, division into the foregoing functional units and modules is merely used as an example for description. In actual application, the foregoing functions can be allocated to different functional units and modules for implementation based on a requirement, that is, an inner structure of the apparatus is divided into different functional units or modules to implement all or some of the functions described above.

The foregoing embodiments are merely intended to describe the technical solutions of this application, but are not to limit this application. Although this application is described in detail with reference to the foregoing embodiments, a person of ordinary skill in the art should understand that the person of ordinary skill in the art may still make modifications to the technical solutions described in the foregoing embodiments or make equivalent replacements to some technical features thereof, without departing from the spirit and scope of the technical solutions of embodiments of this application, and these modifications and replacements shall fall within the protection scope of this application.

## Claims

1. An adaptive action evaluation method, applied to an electronic device with a screen, wherein an action is displayed on the screen, and the method comprises:
obtaining a basic information parameter of a user;
obtaining a first evaluation threshold based on the basic information parameter, wherein the first evaluation threshold is used to provide an evaluation standard;
obtaining a key evaluation parameter of a current action of the user;
determining an action completion evaluation of the user based on the key evaluation parameter and the first evaluation threshold; and
adjusting the first evaluation threshold based on the action completion evaluation.

2. The method according to claim 1, wherein after the obtaining a first evaluation threshold based on the basic information parameter, the method further comprises:
obtaining an evaluation parameter of a warm-up action of the user;
determining a warm-up completion degree of the user based on the evaluation parameter; and
pre-adjusting the first evaluation threshold based on the warm-up completion degree.

3. The method according to claim 1, wherein the obtaining a basic information parameter of a user comprises:
initiating a request for searching for a cloud or an electronic device of an associated account;
receiving a reply from the cloud or the electronic device of the associated account, and determining that the cloud or the electronic device of the associated account exists;
initiating an invoking request for the basic information parameter; and
obtaining the basic information parameter sent by the cloud or the electronic device of the associated account.

4. The method according to claim 1, wherein the obtaining a first evaluation threshold based on the basic information parameter comprises:
classifying the basic information parameter;
assigning a first weight to basic information parameters of a same category, and performing weighted calculation based on the basic information parameters of the same category, to obtain an evaluation result of the category; and
assigning a second weight to basic information parameters of different categories, and performing weighting based on evaluation results of the different categories to obtain the first evaluation threshold.

5. The method according to claim 1, wherein the obtaining a key evaluation parameter of a current action of the user comprises:
determining a type of the key evaluation parameter of the current action;
starting a corresponding monitoring module based on the type of the key evaluation parameter of the current action; and
obtaining the key evaluation parameter of the current action by using the monitoring module.

6. The method according to claim 5, wherein the monitoring module comprises a photographing module and a timing module, and the obtaining a key evaluation parameter of a current action of the user comprises:
determining the type of the key evaluation parameter of the current action;
if the type of the key evaluation parameter is an action angle, starting the photographing module, and obtaining the action angle of the current action by using the photographing module; and
if the type of the key evaluation parameter is duration, starting the photographing module and the timing module, and obtaining the duration of the current action by using the photographing module and the timing module.

7. The method according to claim 1, wherein the determining an action completion evaluation of the user based on the key evaluation parameter and the first evaluation threshold comprises:
classifying the basic information parameter based on a preset parameter classification standard; and
determining the action completion evaluation based on the first evaluation threshold, the classified basic information parameter, and a preset evaluation table, wherein the evaluation table stores mapping information between the basic information parameter and the action completion evaluation.

8. The method according to claim 1, wherein the adjusting the first evaluation threshold based on the action completion evaluation comprises:
determining whether the action completion evaluation meets a preset threshold adjustment condition, wherein there are one or more threshold adjustment conditions; and
adjusting the first evaluation threshold based on the threshold adjustment condition if the threshold adjustment condition is met.

9. The method according to any one of claims 1 to 8, wherein the basic information parameter comprises a basic body parameter, an exercise purpose, and a previous training experience, or comprises a parameter obtained through physical fitness evaluation.

10. An electronic device with a screen, comprising a memory, a processor, and a computer program that is stored in the memory and that can be run on the processor, wherein when executing the computer program, the processor implements the following steps:
obtaining a basic information parameter of a user;
obtaining a first evaluation threshold based on the basic information parameter, wherein the first evaluation threshold is used to provide an evaluation standard;
obtaining a key evaluation parameter of a current action of the user;
determining an action completion evaluation of the user based on the key evaluation parameter and the first evaluation threshold; and
adjusting the first evaluation threshold based on the action completion evaluation.

11. The electronic device according to claim 10, wherein the processor executes the computer program, and further implements the following steps:
obtaining an evaluation parameter of a warm-up action of the user;
determining a warm-up completion degree of the user based on the evaluation parameter; and
pre-adjusting the first evaluation threshold based on the warm-up completion degree.

12. The electronic device according to claim 10, wherein when the processor executes the computer program to implement the obtaining a basic information parameter of a user, the electronic device comprises:
initiating a request for searching for a cloud or an electronic device of an associated account;
receiving a reply from the cloud or the electronic device of the associated account, and determining that the cloud or the electronic device of the associated account exists;
initiating an invoking request for the basic information parameter; and
obtaining the basic information parameter sent by the cloud or the electronic device of the associated account.

13. The electronic device according to claim 10, wherein when the processor executes the computer program to implement the obtaining a first evaluation threshold based on the basic information parameter, the electronic device comprises:
classifying the basic information parameter;
assigning a first weight to the basic information parameters of a same category, and performing weighted calculation based on the basic information parameters of the same category, to obtain an evaluation result of the category; and
assigning a second weight to the basic information parameters of different categories, and performing weighting based on the evaluation results of the different categories to obtain the first evaluation threshold.

14. The electronic device according to claim 10, wherein when the processor executes the computer program to implement the obtaining the key evaluation parameter of the current action, the electronic device comprises:
determining a type of the key evaluation parameter of the current action;
starting a corresponding monitoring module based on the type of the key evaluation parameter of the current action; and
obtaining the key evaluation parameter of the current action by using the monitoring module.

15. The electronic device according to claim 14, wherein the monitoring module comprises a photographing module and a timing module, and when the processor executes the computer program to implement the obtaining a key evaluation parameter of a current action of the user, the electronic device comprises:
determining a type of the key evaluation parameter of the current action;
if the type of the key evaluation parameter is an action angle, starting the photographing module, and obtaining the action angle of the current action by using the photographing module; and
if the type of the key evaluation parameter is duration, starting the photographing module and the timing module, and obtaining the duration of the current action by using the photographing module and the timing module.

16. The electronic device according to claim 10, wherein when the processor executes the computer program to implement the determining an action completion evaluation of the user based on the key evaluation parameter and the first evaluation threshold, the electronic device comprises:
classifying the basic information parameter based on a preset parameter classification standard; and
determining the action completion evaluation based on the first evaluation threshold, the classified basic information parameter, and a preset evaluation table, wherein the evaluation table stores mapping information between the basic information parameter and the action completion evaluation.

17. The electronic device according to claim 10, wherein when the processor executes the computer program to implement the adjusting the first evaluation threshold based on the action completion evaluation, the electronic device comprises:
determining whether the action completion evaluation meets a preset threshold adjustment condition, wherein there are one or more threshold adjustment conditions; and
adjusting the first evaluation threshold based on the threshold adjustment condition if the threshold adjustment condition is met.

18. The electronic device according to any one of claims 10 to 17, wherein the basic information parameter comprises a basic body parameter, an exercise purpose, and a previous training experience, or comprise parameters obtained through physical fitness evaluation.

19. A computer-readable storage medium, storing a computer program, wherein when the computer program is executed by a processor, the steps of the adaptive action evaluation method according to any one of claims 1 to 9 are implemented.
